Europäisches Patentamt

European Patent Office

Office européen des brevets

(19)

(11) **EP 0 662 835 B1**

(12) **FASCICULE DE BREVET EUROPEEN**

(45) Date de publication et mention
de la délivrance du brevet:
**27.11.1996 Bulletin 1996/48**

(21) Numéro de dépôt: **93920939.1**

(22) Date de dépôt: **28.09.1993**

(51) Int. Cl.$^6$: **A61K 31/71**, A61K 47/22

(86) Numéro de dépôt international:
**PCT/FR93/00945**

(87) Numéro de publication internationale:
**WO 94/07504 (14.04.1994 Gazette 1994/09)**

(54) **NOUVELLES FORMULATIONS PHARMACEUTIQUES DE SPIRAMYCINE**

NEUE PHARMAZEUTISCHE FORMULIERUNG ENTHALTEND SPIRAMYCINE

NEW PHARMACEUTICAL FORMULATIONS OF SPIRAMYCINE

(84) Etats contractants désignés:
**AT BE CH DE DK ES FR GB GR IE IT LI LU NL PT
SE**

(30) Priorité: **05.10.1992 FR 9211747**

(43) Date de publication de la demande:
**19.07.1995 Bulletin 1995/29**

(73) Titulaire: **RHONE-POULENC RORER S.A.
92160 Antony (FR)**

(72) Inventeurs:
• **PIOT, François-Xavier
F-94320 Thiais (FR)**
• **RONA, Robert
F-78100 Saint Germain-en-Laye (FR)**

(74) Mandataire: **Savina, Jacques et al
RHONE-POULENC SANTE,
Service Brevets,
20 Avenue Raymond Aron
92165 Antony Cédex (FR)**

(56) Documents cités:
**EP-A- 0 396 165          WO-A-92/09269**

• **CHEMICAL ABSTRACTS, vol. 115, no. 6, 12 Août
1991, Columbus, Ohio, US; abstract no. 57213u,
ALVAREZ DEL RIO ET AL 'Water-dispersible
spiramycin formulations'**
• **O BRIEN NABORS ET AL 'Alternative
sweeteners' 1986 , M. DEKKER , NEW YORK US
voir page 2 - page 3 voir page 96 - page 99**

**Description**

La présente invention concerne de nouvelles formulations pharmaceutiques de spiramycine. Elle concerne plus particulièrement de nouvelles formulations destinées à être administrées par voie orale.

Depuis de nombreuses années, il est connu que la spiramycine est très difficile à administrer à l'homme et plus particulièrement à l'enfant sous forme de solution, suspension ou granulé dispersible car elle présente une amertume extrêmement difficile à masquer.

Il a été tenté de masquer cette amertume par exemple dans la formulation décrite dans la demande de brevet publiée sous le numéro FR 2 669 533. Dans cette formulation, la spiramycine est encapsulée par de l'albumine par une technique qui exige la mise en oeuvre de solvants organiques tels que l'isooctane et leur élimination en fin de procédé. Cette technique, bien que très performante, est très coûteuse car elle ne permet que la fabrication de quantités réduites de composition pharmaceutique et elle exige des étapes de recyclage de solvants longues et onéreuses.

La présente invention a permis de préparer des formulations de spiramycine ayant un goût amélioré sans utiliser de solvant pour sa préparation.

Elle consiste à utiliser un édulcorant qui masque l'amertume de la spiramycine. Cet édulcorant est l'acésulfame de potassium.

La présente invention concerne donc de nouvelles formes pharmaceutiques de spiramycine à base de spiramycine, d'acésulfame et d'agents aromatisants destinées à être administrées sous forme de doses (sachets, flacons ou boites avec une mesure) contenant une poudre granulée à dissoudre ou à disperser dans l'eau.

Cette nouvelle forme granulée qui peut être mise en suspension extemporanée dans l'eau offre les avantages suivants :

- facilité d'emploi en traitement ambulatoire
- précision du dosage unitaire
- mise en suspension ou en solution facile dans l'eau
- absorption facile.

De nombreux essais de formulations diverses essayant de masquer l'amertume ont été entrepris. Aucun n'a donné de résultats satisfaisants quant au goût de la suspension aqueuse obtenue ou quant à la biodisponibilité de la spiramycine après absorption. L'association entre l'acésulfame de potassium et la spiramycine a permis d'atteindre cet objectif.

Le procédé de préparation des nouvelles formes pharmaceutiques granulées de la spiramycine pour administration orale consiste à réaliser une granulation humide de la spiramycine et du saccharose, de préférence dans un rapport pondéral entre 1/1 à 1/9, puis à réaliser à sec un mélange des granulés obtenus précédemment, de l'acésulfame, des aromes et du reste du saccharose.

Les nouvelles formulations selon l'invention contiennent de préférence :

| - spiramycine | 100 000 à 5 000 000 UI |
|---|---|
| - acésulfame de potassium | 10 à 20 mg |
| - aromes | 20 à 200 mg |
| -saccharose | qsp 1 à 10 g |

Elles contiennent encore plus préférentiellement :

| - spiramycine | 375 000 UI |
|---|---|
| - acésulfame de potassium | 10 à 20 mg |
| - aromes | 60 mg |
| - saccharose | qsp 3 g |

La composition ci-dessus n'est que préférée, elle sera adaptée en fonction du masquage désiré de l'amertume du principe actif par addition de quantité supplémentaire d'acésulfame de potassium. Les arômes seront adaptés au goût et à l'âge de l'enfant.

Ces formulations peuvent être indifféremment présentées sous forme de doses comme mentionné précédemment ou sous forme de solution ou suspension extemporanée.

L'invention sera plus complètement décrite à l'aide des exemples suivants.

**ESSAI COMPARATIF 1**

| | |
|---|---|
| - Spiramycine | 86,190 mg (375 000 UI) |
| - Eudragit E 100 ® | 70,00 mg |
| - Mannitol q.s.p. | 950,00 mg |
| - Saccharinate de sodium | 25,00 mg |
| - Arôme de fraise | 40,00 mg |
| - Silice colloïdale anhydre | 12,50 mg |
| - Cellulose (microcristalline) | 25,00 mg |
| - Polyvidone | 100,00 mg |
| - Saccharose | 97,50 mg |
| | 1250,00 mg |

Les essais avec ce type de formule ont été arrêtés, malgré la réussite du masquage du goût, en raison du mauvais résultat de biodisponibilité.

**ESSAI COMPARATIF 2**

Mise au point d'une formule simple et rationnelle

- préparation d'un granulé (concentré) composé de sucre et de la spiramycine
- aromatisation du granulé primaire par l'addition en phase externe des édulcorants et des aromatisants par mélange simple.

La fabrication du granulé primaire a été élaboré dans un mélangeur-granuleur-sécheur Turbosphère et la préparation du mélange final dans un mélangeur à gravité.

| Formule unitaire théorique : | |
|---|---|
| - spiramycine base | 375 000 UI (81,156 mg) |
| - saccharose q.s.p. | 1000,00 mg |
| - saccharose (Alveo sucre) | 1960,000 mg) phase |
| - arôme banane | 40,000 mg) externe |
| | 3000,000 mg |

L'étude de biodisponibilité a démontré la bioéquivalence du granulé de l'essai comparatif 2 et du sirop commercial cependant les tests d'acceptabilité du goût ont montré que l'aromatisation du produit nécessite une amélioration.

**ESSAIS COMPARATIFS 3 ET 4**

Les édulcorants couramment employés (saccharinate de sodium, cyclamate de sodium) n'ont pas pu être retenus en raison de l'insuffisance de l'effet organoleptique.

Un test d'acceptabilité a été réalisé sur deux de ces formulations (une avec saccharinate de scdium et une avec aspartam). Les résultats sont indiqués dans le tableau ci-après. Pour l'aspartam le test d'acceptabilité était satisfaisant mais le produit interagit avec la spiramycine ce qui rend son utilisation impossible.

**ESSAI COMPARATIF 5**

On a essayé de masquer l'amertume de la spiramycine par association avec de la gomme xanthane, les résultats des tests de dégustation n'ont pas été concluants.

| | ESSAIS COMPARATIFS 2 A 5 | | | | INVENTION | |
|---|---|---|---|---|---|---|
| | $C_5$ | $C_2$ | $C_4$ | $C_3$ | 1 | 2 |
| Spiramycine | 375 000 UI | 375 000 UI | 375 000 UI | 375 000UI | 375 000 UI | 375 000 UI |
| Saccharose | | qsp 1 g | | qsp 1 g | qsp 1 g | qsp 165 mg |
| Amidon | 3,5 mg | | | | | |
| Gomme Xanthane | 15 mg | | | | | |
| P.V.P. | | | 100 mg | | | |
| Acésulfame de K | | | | | 20 mg | 10 mg |
| Aspartam | | | | 10 mg | | |
| Saccharinate | | | 25 mg | | | |
| Eudragit E® | | | 70 mg | | | |
| Mannitol | | | 800 mg | | | |
| Arômes | 20 mg | 40 mg | 40 mg | 20 mg | 60 mg | 60 mg |
| Saccharose | qsp 2,5 g | qsp 3 g | qsp 1,25 | qsp 3 g | qsp 3 g | qsp 3 g |
| Notes sur 20 | 6,25 | mauvais | | 14 | 15 | acceptable |
| Observations | | | non bioéquivalent | interaction entre la spiramycine et l'aspartam | | |

**ESSAIS SELON L'INVENTION**

La fabrication des sachets se déroule en 3 phases :

a - Préparation d'un granulé primaire concentré (165 mg par sachet de 3 g renfermant 375 000 UI de spiramycine) dans un mélangeur-granulateur-sécheur Moritz :

| par sachet de 3 g | |
|---|---|
| - spiramycine base | 375 000 UI (84,081 mg) |
| - saccharose (sucre surfin) q.s.p. | 165,00 mg |
| - eau | environ 5 % en masse |

Pendant la granulation , on chauffe le produit à l'aide d'une double enveloppe jusqu'à environ 55°C. On agite à environ 100 tours/minute pendant 30 minutes. Pour le séchage, on agite à environ 20 tours/minute en maintenant la température mais en baissant la pression entre 6 et 20 kPa pendant 60 minutes.

On refroidit ensuite le produit à température ambiante en 30 minutes environ.

On tamise le granulé sur une grille de 0,71 mm d'ouverture.

b - Préparation du granulé final dans un mélangeur cubique à gravité

| - granulé primaire | 165,00 mg |
|---|---|
| - acésulfame de potassium | 10,00 mg |
| - arôme fraise en poudre | 30,00 mg |
| - arôme framboise en poudre | 30,00 mg |
| - saccharose* qsp | 3000,00 mg |

\* sucre calibré surfin et Alveo-sucre en proportion 1/1 environ.

c - Répartition du mélange final à raison de :

- 3 g pour le dosage de 375 000 UI
- 6 g pour le dosage de 750 000 UI
- 12 g pour le dosage de 1 500 000 UI

par sachet de complexe papier/aluminium/polyéthylène

Cette formulation présente une nette amélioration par rapport au formulations de l'art antérieur quant au masquage du goût et de l'arrière goût de la spiramycine.

**Revendications**

1. Nouvelles formulations de spiramycine destinées à être administrées par voie orale caractérisées en ce qu'elles contiennent une association entre la spiramycine et l'acésulfame de potassium.

2. Nouvelles formulations selon la revendication 1 caractérisées en ce qu'elles se présentent sous forme granulée destinées à être mise en suspension ou en solution dans l'eau.

3. Nouvelles formulations selon l'une quelconque des revendications précédentes caractérisées en ce qu'elles contiennent :

| - spiramycine | 100 000 à 5 000 000 UI |
|---|---|
| - acésulfame de potassium | 10 à 20 mg |
| - arômes | 20 à 200 mg |
| - saccharose | qsp 1 à 10 g |

4. Nouvelles formulations selon la revendication 3 caractérisées en ce qu'elles contiennent :

| - spiramycine | 375 000 UI |
|---|---|
| - acésulfame de potassium | 10 à 20 mg |
| - arômes | 60 mg |
| -saccharose | qsp 3 g |

5. Procédé de préparation des nouvelles formulations de spiramycine selon l'une quelconque des revendications précédentes caractérisé en ce qu'on réalise une granulation humide de la spiramycine et d'une partie ou la totalité de saccharose puis on mélange à sec l'acésulfame, les arômes et le reste du saccharose.

**Claims**

1. New spiramycin formulations intended to be administered orally, characterized in that they contain an association between spiramycin and potassium acesulfame.

2. New formulations according to claim 1, characterized in that they are provided in granulated form, intended to be suspended or dissolved in water.

3. New formulations according to any one of the preceding claims, characterized in that they contain:

| - spiramycin | 100,000 to 5,000,000 IU |
|---|---|
| - potassium acesulfame | 10 to 20 mg |
| - flavourings | 20 to 200 mg |
| - sucrose | qs 1 to 10 g. |

4. New formulations according to claim 3, characterized in that they contain:

| - spiramycin | 375,000 IU |
|---|---|
| - potassium acesulfame | 10 to 20 mg |
| - flavourings | 60 mg |
| - sucrose | qs 3 g. |

5. Process for preparing new spiramycin formulations according to any one of the preceding claims, characterized in that the spiramycin and a portion or all of the sucrose are subjected to wet granulation and then the acesulfame, the flavourings and the remainder of the sucrose are mixed dry.

**Patentansprüche**

1. Neue Spiramycinformulierungen zur Verabreichung auf oralem Weg, dadurch **gekennzeichnet**, daß sie eine Assoziation aus Spiramycin und Kaliumacesulfam enthalten.

2. Neue Formulierungen nach Anspruch 1, dadurch **gekennzeichnet**, daß sie in granulierter Form zur Lösung oder Suspendierung in Wasser vorliegen.

3.  Neue Formulierungen nach einem der vorstehenden Ansprüche, dadurch **gekennzeichnet**, daß sie enthalten:

| Spiramycin | 100 000 bis 5 000 000 IE |
|---|---|
| Kaliumacesulfam | 10 bis 20 mg |
| Geschmacksstoffe | 20 bis 200 mg |
| Saccharose | q.s. für 1 - 10 g |

4.  Neue Formulierungen nach Anspruch 3, dadurch **gekennzeichnet**, daß sie enthalten:

| Spiramycin | 375 000 IE |
|---|---|
| Kaliumacesulfam | 10 bis 20 mg |
| Geschmacksstoffe | 60 mg |
| Saccharose | q.s. für 3 g |

5.  Verfahren zur Herstellung von neuen Spiramycinformulierungen nach einem der vorstehenden Ansprüche, dadurch **gekennzeichnet**, daß man eine Feuchtgranulierung von Spiramycin und einem Teil oder der Gesamtmenge der Saccharose durchführt, dann Acesulfam, Geschmacksstoffe und den Rest der Saccharose trocken dazumischt.